# EUROPÄISCHE PATENTSCHRIFT

(11) **EP 0 541 507 B1**
(45) Veröffentlichungstag und Bekanntmachung des Hinweises auf die Patenterteilung: **10.10.2001**
(21) Anmeldenummer: 92890232.9
(22) Anmeldetag: 02.11.1992
(51) Int. Cl.: C12N 9/74, A61K 38/46

(54) **Thrombin sowie Verfahren zu seiner Herstellung**
Thrombin and method for its production
Thrombine et procédé pour sa production

(30) Priorität: 04.11.1991 AT 218391
(43) Veröffentlichungstag der Anmeldung: 12.05.1993
(73) Patentinhaber: Baxter Aktiengesellschaft, 1221 Wien (AT)
(72) Erfinder: Eibl, Johann, Dr., A-1180 Wien (AT); Linnau, Yendra, Dr., A-1224 Wien (AT)
(74) Vertreter: Alge, Daniel, Mag. Dr. rer.nat.

(56) Entgegenhaltungen:
- EP-A- 0 041 173
- EP-A- 0 378 798
- EP-A- 0 443 724
- WO-A-82/03871
- DE-A- 3 809 991

## Beschreibung

Die Erfindung betrifft ein Thrombin menschlichen oder tierischen Ursprungs sowie ein Verfahren zu seiner Herstellung und dessen Verwendung.

Die Blutgerinnung durchläuft eine Serie von aufeinanderfolgenden Reaktionen, in denen Blutgerinnungsfaktoren aktiviert werden und letztlich Fibrin durch die Wirkung von aktiviertem Prothrombin (Thrombin) auf Fibrinogen gebildet wird. Die Umwandlung von Prothrombin in Thrombin verläuft mit Faktor Xa und Calcium allein sehr langsam. Sie verläuft erst dann optimal, wenn wiederum ein Komplex mehrerer Faktoren (Prothrombinase-Komplex) vorliegt. Neben Faktor Xa gehören zu diesem Komplex Faktor V, Phospholipide und Calcium. Der Faktor Xa spaltet das Prothrombinmolekül (Molekulargewicht 68 kD) proteolytisch und generiert somit das aktive Enzym Thrombin (Molekulargewicht 30 kD).

Die Plasmaprotease Thrombin ist ein multifunktionelles Enzym, welches nicht nur durch Spaltung des Fibrinogens zu Fibrin koagulierend wirkt, sondern auch die Gerinnungsfaktoren V, VIII und XIII aktiviert und sein eigenes Proenzym (Prothrombin) spaltet.

In der Therapie wird Thrombin allein oder gemeinsam mit Fibrinogen zur Stillung von Blutungen oder in der Chirurgie zur Gewebeklebung eingesetzt.

Die Aktivierung von Prothrombin via dem Prothrombinasekomplex ist ex situ schwer nachzuahmen, weshalb es nicht an Versuchen fehlt, Thrombin durch die Einwirkung von Proteasen menschlichen oder tierischen Ursprungs zu generieren. Dabei ist zu bedenken, daß jeder Kontakt des Produktes mit menschlichem oder tierischem Material wegen der Kontaminationsgefahr mit infektiösen Agentien zu vermeiden ist.

Versuche zur Behandlung eines Prothrombinkomplexes, isoliert aus Plasma, mit Calciumionen sowie mit Calciumionen und einer Suspension enthaltend bovines Thromboplastin zeigten, daß die Behandlung mit Calciumionen alleine zu einer wesentlich geringeren Ausbeute und Reinheit des gebildeten Thrombins führt als die Behandlung mit Calciumionen und Thromboplastin (Cryobiology 21, 661-663 (1984)).

Aus der DE-A - 38 43 126 ist bekannt, daß aus Plasma, welches an eine Matrix adsorbiert und mit einem Prothrombinaktivator behandelt ist, Thrombin gewonnen werden kann. Als Aktivator werden beispielsweise Calciumionen, Calciumionen und Thromboplastin oder Faktor Xa genannt. Während der Aktivierung sind sämtliche biologische Cofaktoren, welche ebenfalls an der Matrix gebunden werden, anwesend.

Bei der Verwendung von plasmatischem Prothrombin zur Gewinnung von Thrombin besteht die Gefahr einer Kontamination mit infektiösen Agentien (z.B. Hepatitis-Viren; HIV). Dazu kommt noch, daß bei allen bekannten Aktivierungsverfahren neben Calcium²⁺ -Ionen die zusätzliche Verwendung biologischer Co-Faktoren empfohlen wird, womit eine weitere Kontaminationsquelle gegeben ist.

Nun ist zwar bekannt, daß infektiöse Agentien in biologischen Präparaten zuverlässig durch eine Hitzebehandlung, insbesondere in Kombination mit einer Dampfbehandlung, inaktiviert werden können (AT-B - 385.657). Es hat sich aber gezeigt, daß Thrombin aufgrund seiner Hitzelabilität in Gegenwart von Stabilisatoren erhitzt werden muß (DE-A - 38 09 991), um die Aktivität des Thrombins nicht zu beeinträchtigen. Die Verwendung von Stabilisatoren ist jedoch umstritten, da während der Hitzebehandlung nicht nur die Thrombinaktivität geschützt wird, sondern auch Viren stabilisiert werden.

Die Erfindung stellt sich die Aufgabe, ein virussicheres Thrombin zur Verfügung zu stellen.

Die erfindungsgemäße pharmazeutische Präparation enthaltend virussicheres Thrombin, dem ein gerinnungsaktives Salz zugesetzt ist, ist erhältlich aus einer virusinaktivierten Prothrombin-haltigen Plasmafraktion durch Aktivierung mit dem gerinnungsaktiven Salz, beispielsweise mit Calcium-, Strontium- oder Zinkionen, jedoch ohne Zugabe von Phospholipiden. Derartige Salze fördern die Bildung von Thrombin aus den entsprechenden Gerinnungsfaktoren.

Die Erfindung beruht auf der Erkenntnis; daß infektiöse Agentien, welche in plasmatischem Prothrombin vorhanden sind, durch eine Behandlung des Prothrombins zur Virusinaktivierung unschädlich gemacht werden können, ohne die biologische Aktivität des aus dem Prothrombin erhältlichen Thrombins wesentlich zu beeinträchtigen.

Es hat sich überraschenderweise gezeigt, daß die Aktivierung einer virusinaktivierten prothrombin-haltigen Fraktion allein durch Zugabe von gerinnungsaktiven Salzen in einfacher Weise zu einer hohen Ausbeute bzw. Reinheit des Produktes führt, ohne weitere Zugabe von biologischen Co-Faktoren, wie die Gerinnungsfaktoren V, Xa oder Phospholipide. Eine Kontamination während der Aktivierung wird also vermieden, weshalb auch das generierte Thrombin, gleich dem Ausgangsmaterial, als virussicher gilt.

Es hat sich als vorteilhaft erwiesen, die erfindungsgemäßen Präparate enthaltend virussicheres Thrombin aus virusinaktiviertem Prothrombinkomplex, insbesondere aus virusinaktiviertem aktiviertem Prothrombinkomplex, zu generieren. Die Aktivierung von aktiviertem Prothrombinkomplex durch Zusatz von gerinnungsaktiven Salzen erfolgt mit überraschend hoher Reaktionsgeschwindigkeit. Die Ausbeute an Thrombin wird gleichfalls optimiert.

Besonders vorteilhaft ist die Herstellung der Präparation enthaltend virussicheres Thrombin aus FEIBA. Ein aktivierter Prothrombinkomplex bzw. FEIBA kann durch bereits bekannte Maßnahmen (AT-B-350 726, AT-B-368 883, EP-B-0 041 173) aus einem Prothrombinkomplex hergestellt werden.

Die Erfindung betrifft auch ein Verfahren zur Herstellung einer virussicheren Thrombin-Präparation und ist gekennzeichnet durch die Kombination der Maßnahmen, daß
- ein aktivierter Prothrombinkomplex aus einer prothrombinhältigen Plasmafraktion hergestellt wird,
- der aktivierte Prothrombinkomplex zur Inaktivierung infektiöser Agentien behandelt wird, und
- gerinnungsaktive Salze zum behandelten aktivierten Prothrombinkomplex zugesetzt werden, um Thrombin zu generieren, wobei kein Thromboplastin zugesetzt wird.

Die erfindungsgemäße Thrombin-Präparation wird vorteilhafterweise durch Ionenaustauscherchromatographie und/oder Affinitätschromatographie weiter gereinigt.

Eine virussichere Thrombin-Präparation gemäß der obigen Beschreibung eignet sich besonders zur Verwendung von pharmazeutischen Präparationen und zur Herstellung von Diagnostika.

Mit den nachfolgenden Beispielen wird die Erfindung noch näher erläutert, wobei die Beispiele 3 und 4 die weitere Reinigung des nach Beispiel 1 hergestellten Thrombins betreffen.

### Beispiel 1:

Aus 15 l menschlichem kryopräzipitat-armen Blutplasma wurden Prothrombin (Faktor II) neben den Gerinnungsfaktoren VII, IX und X an einem Anionenaustauscher (DEAE-Sephadex) gebunden. Nach der Elution der Faktor II-enthaltenden Fraktion mittels 0,5-molarer NaCl-Lösung wurde bei dieser Lösung die Salzkonzentration auf 0,15 Mol/l durch Diafiltration verringert und anschließend gefriergetrocknet.

Um eventuell enthaltene Krankheitserreger zu inaktivieren, wurde diese Fraktion nach der AT-B - 385.657 10 h bei 60°C und 1 h bei 80°C erhitzt. Die Prothrombinaktivität betrug 5.250 E. Das Prothrombin wurde in einer Lösung zu 2,5 E/ml gelöst und mit 2,5 mMol/l CaCl₂ bei +30°C bei einem pH-Wert von 7,0 langsam gerührt; nach 80 min wurde die Thrombinaktivität (mittels chromogenem Substrat Th-1 (Fa. Immuno)) mit 48 E pro 1 E Faktor II bestimmt.

Durch Abkühlung auf +4°C und durch Zusatz von Ethylendiaminotetraessigsäure (EDTA) wurde die Thrombin-Generierung gestoppt. Mittels Ultrafiltration/Diafiltration mit einer Ultrafiltrationsmembran (Porengröße: 10.000) wurde der Ca-Komplex entfernt. Anschließend wurde das Konzentrat zu einer pharmazeutischen Präparation fertiggestellt.

### Beispiel 2:

20 ml einer FEIBA-hältigen Lösung (IMMUNO AG, Wien) mit einer FEIB-Aktivität von 966 Einheiten und 992 Einheiten Faktor II wurde mit einer 0,9 %igen NaCl-Lösung auf 330 ml verdünnt und mit 2,75 mMol/l CaCl₂ bei +30°C langsam gerührt. Nach 90 min erreichte die Thrombinaktivität ein Maximum von 51 Einheiten pro Einheit Faktor II. Die Aktivierung wurde durch Abkühlung der Lösung auf +4°C und Zusatz von Natriumcitrat gestoppt.

### Beispiel 3 :

20.000 E Thrombin, nach Beispiel 1 hergestellt, wurden an einer Säule von 20 ml S-Sepharose bei einer Leitfähigkeit von 10,5 mS/cm bei einem pH-Wert von 6,0 adsorbiert. Anschließend wurde mit 140 ml einer 150-mMolaren NaCl-Lösung gewaschen, um die nicht gebundenen Proteine zu entfernen.

Die Thrombin-hältige Fraktion wurde mit 100 ml einer 750-mMolaren NaCl-Lösung eluiert, aufkonzentriert, diafiltriert und schließlich zu einer pharmazeutischen Präparation fertiggestellt.

Die Ausbeute an Thrombinaktivität war mehr als 90 %.

### Beispiel 4:

10.000 E Thrombin, nach Beispiel 1 hergestellt, wurden an einer Säule von 10 ml Lysin-Sepharose, äquilibriert mit einer 150 mMol Natriumacetatlösung, pH 6,7, aufgetragen. Die beladene Säule wurde mit dem gleichen Puffer gewaschen und die thrombinhältige Fraktion wurde mit einer 300 mMolaren Lysin-Lösung eluiert; die Thrombinaktivität war 9400 E und die spezifische Aktivität 1850 E/mg Protein.

## Patentansprüche

1. Pharmazeutische Präparation enthaltend virussicheres Thrombin, dem ein gerinnungsaktives Salz zugesetzt ist, erhältlich aus einer virusinaktivierten Prothrombin-haltigen Plasmafraktion durch Aktivierung mit dem gerinnungsaktiven Salz ohne Zugabe von Phospholipiden.

2. Präparation nach Anspruch 1, erhältlich aus einer Plasmafraktion, die den Prothrombinkomplex enthält.

3. Präparation nach Anspruch 1, erhältlich aus einer Plasmafraktion, die den aktivierten Prothrombinkomplex enthält.

4. Präparation nach Anspruch 1 oder 2, erhältlich aus einer Plasmafraktion, enthaltend FEIBA.

5. Verwendung einer Präparation nach einem der Ansprüche 1 bis 4 zur Herstellung von pharmazeutischen Präparationen und zur Herstellung von Diagnostika.

6. Verfahren zur Herstellung einer virussicheren Thrombin-Präparation, **gekennzeichnet durch** die Kombination der Maßnahmen, daß
- ein aktivierter Prothrombinkomplex aus einer prothrombinhältigen Plasmafraktion hergestellt wird,
- der aktivierte Prothrombinkomplex zur Inaktivierung infektiöser Agentien behandelt wird, und
- gerinnungsaktive Salze zum behandelten aktivierten Prothrombinkomplex zugesetzt werden, um Thrombin zu generieren, wobei kein Thromboplastin zugesetzt wird.

7. Verfahren nach Anspruch 6, **dadurch gekennzeichnet, daß** die Thrombin-Präparation weiters durch Ionenaustauscherchromatographie und/oder Affinitätschromatographie gereinigt wird.

## Claims

1. A pharmaceutical preparation comprising a virus-safe thrombin to which a coagulatively active salt has been added, obtainable from a virus-inactivated prothrombin-containing plasma fraction by activation with the coagulatively active salt without an addition of phospholipids.

2. A preparation according to claim 1, obtainable from a plasma fraction which comprises the prothrombin complex.

3. A preparation according to claim 1, obtainable from a plasma fraction which comprises the activated prothrombin complex.

4. A preparation according to claim 1 or 2, obtainable from a plasma fraction comprising FEIBA.

5. The use of a preparation according to any one of claims 1 to 4 for producing pharmaceutical preparations and for producing diagnostic agents.

6. A method of producing a virus-safe thrombin preparation, **characterised by** the combination of the measures that
· an activated prothrombin complex is prepared from a prothrombin-containing plasma fraction;
· the activated prothrombin complex is treated to inactivate infectious agents, and
· coagulatively active salts are added to the treated activated prothrombin complex to generate thrombin, without any addition of thromboplastin.

7. A method according to claim 6, **characterised in that** the thrombin preparation is further purified by ion exchange chromatography and/or affinity chromatography.

## Revendications

1. Préparation pharmaceutique contenant de la thrombine viralement sûre à laquelle est ajouté un sel actif pour la coagulation, qui peut être obtenue par activation du sel actif pour la coagulation à partir d'une fraction plasmatique contenant de la prothrombine inactivée pour les virus, sans ajout de phospholipides.

2. Préparation selon la revendication 1, qui peut être obtenue à partir d'une fraction plasmatique contenant le complexe de prothrombine.

3. Préparation selon la revendication 1, qui peut être obtenue à partir d'une fraction plasmatique contenant le complexe de prothrombine activé.

4. Préparation selon la revendication 1 ou la revendication 2, qui peut être obtenue à partir d'une fraction plasmatique contenant du FEIBA.

5. Utilisation d'une préparation selon l'une des revendications 1 à 4 pour la fabrication de préparations pharmaceutiques et pour la fabrication de produits de diagnostic.

6. Procédé pour la fabrication d'une préparation de thrombine viralement sûre, **caractérisé par** la combinaison des mesures consistant à :
- préparer un complexe de prothrombine activé à partir d'une fraction plasmatique contenant de la prothrombine,
- traiter le complexe de prothrombine activé afin d'inactiver les agents infectieux, et
- ajouter au complexe de prothrombine activé et traité des sels actifs pour la coagulation afin de générer de la thrombine, sans ajout de thromboplastine.

7. Procédé selon la revendication 6, **caractérisé en ce que** la préparation de thrombine est également purifiée par chromatographie à échange d'ions et/ou par chromatographie d'affinité.
